# EUROPÄISCHE PATENTANMELDUNG

(11) **EP 1 586 607 A1**
(43) Veröffentlichungstag der Anmeldung: **19.10.2005**
(21) Anmeldenummer: 05007822.9
(22) Anmeldetag: 09.04.2005
(51) Int. Cl.: C09C 1/56, C08K 9/04, D01F 11/14, C07D 257/04, C09C 3/08

(54) **Kohlenstoffmaterial**

(30) Priorität: 17.04.2004 DE 102004018746
(71) Anmelder: Degussa AG, 40474 Düsseldorf (DE)
(72) Erfinder: Schukat, Gerd, Dr., 06217 Merseburg (DE); Knackfuss, Bernd, Dr., 04277 Leipzig (DE); Fanghänel, Egon, Prof. Dr., 06114 Halle (DE); Lüthge, Thomas, Dr., 63452 Hanau (DE); McIntosh, Ralph, 63457 Hanau (DE); Bergemann, Klaus, Dr., 50170 Kerpen-Sindorf (DE)

(57) **Zusammenfassung**

Kohlenstoffmaterial mit organischen Gruppen, erhältlich durch Umsetzung von Kohlenstoffmaterial mit organischen Verbindungen der allgemeinen Formel 1

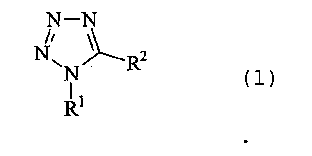

Die erfindungsgemäßen Kohlenstoffmaterialien mit organischen Gruppen können als Füllstoffe, Verstärkerfüllstoffe, UV-Stabilisatoren, Leitfähigkeitsruße oder Pigment verwendet werden.

## Beschreibung

Die Erfindung betrifft ein Kohlenstoffmaterial, ein Verfahren zu seiner Herstellung sowie seine Verwendung.

Aus EP 0569503 ist ein Verfahren zur Oberflächenmodifizierung von Kohlenstoffmaterialien mit aromatischen Gruppen durch elektrochemische Reduktion eines Diazoniumsalzes bekannt.

Weiterhin ist bekannt, Kohlenstoffmaterialien mit organischen Gruppen zu versehen, indem man die organischen Gruppen über eine Diazotierung mit dem Kohlenstoffmaterial verknüpft (WO 96/18690), die organischen Gruppen mittels Umsetzungen mit Radikalbildnern (Ohkita K., Tsubokawa N., Saitoh E., Carbon 16 (1978) 41, DE 10012784.3) oder über Cycloadditionen (DE 10012783.5, JP 11315220 A) an das Kohlenstoffmaterial bindet.

Die bekannten Verfahren haben folgende Nachteile:
- Die, neben dem giftigen und brandfördernden Natriumnitrit, ebenfalls zur Diazotierung verwendbaren nichtionischen organischen Nitrite sind giftig und leicht brennbar. Reste der Nitrite (Gegenionen, Alkylreste) verbleiben ungebunden als Verunreinigung im Kohlenstoffmaterial.
- Zur Durchführung der Diazotierung ist der Einsatz von Nitrit im sauren Medium erforderlich. Dabei können sich giftige Stickstoffoxide bilden.
- Radikalbildner sind thermisch beziehungsweise photochemisch labil, explosionsgefährlich und können zu schwer beherrschbaren Kettenreaktionen führen.
- Die Synthese und Reinigung der entsprechenden Vorstufen der Radikalbildner laufen zum Teil über giftige beziehungsweise geruchsbelästigende Stoffe.

Aufgabe der Erfindung ist es, ein Kohlenstoffmaterial mit organischen Gruppen zur Verfügung zu stellen, wobei
- die Modifizierung des Kohlenstoffmaterials so variabel ist, dass die Eigenschaften des Kohlenstoffmaterials beeinflussenden Gruppen nahe an der Oberfläche des Kohlenstoffmaterials und/oder auch weit von der Oberfläche des Kohlenstoffmaterials entfernt angeordnet sein können,
- die Modifizierung des Kohlenstoffmaterials ohne vorgelagerte Reaktionen, wie Aktivierung durch Starter, abläuft,
- die Umsetzungen mit den erfindungsgemäß verwendeten Modifizierungsmitteln thermisch ablaufen und zusätzliche Katalysatoren (zum Beispiel Lewissäuren) nicht benötigt werden,
- auf Grund der chemischen Eigenschaften der erfindungsgemäß verwendeten Modifizierungsmittel keine störenden Nebenreaktionen oder schwer zu kontrollierende Kettenreaktionen ablaufen können,
- das resultierende Kohlenstoffmaterial nicht durch anorganische Säuren bzw. Salze und ähnliches verunreinigt ist, so dass keine Reinigung des Kohlenstoffmaterials erforderlich ist,
- das Kohlenstoffmaterial nicht mit hohem Energieaufwand getrocknet werden muß,
- keine giftigen Abgase bei der Modifizierung entstehen,
- keine oder nur geringe Mengen Lösungsmittel erforderlich sind, die ausserdem leicht entfernbar sind,
- keine giftigen und brandfördernden Alkalinitrite, oder ebenfalls zur Diazotierung verwendbare nichtionische organische Nitrite, die giftig und leicht brennbar sind, verwendet werden und somit keine Reste der Nitrite (Gegenionen, Alkylreste) als Verunreinigung ungebunden im Kohlenstoffmaterial verbleiben und
- keine Radikalbildner verwendet werden, die thermisch beziehungsweise photochemisch labil, und explosionsgefährlich sein können und zu schwer beherrschbaren Kettenreaktionen führen können.

Gegenstand der Erfindung ist ein Kohlenstoffmaterial mit organischen Gruppen, welches dadurch gekennzeichnet ist, dass dieses erhältlich ist durch die Umsetzung von Kohlenstoffmaterial mit organischen Verbindungen der allgemeinen Formel 1, 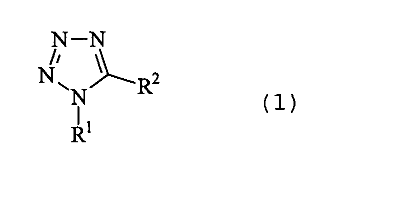 wobei R¹ und R² gleich oder verschieden sind und aus H, Akzeptorgruppen, Donatorgruppen, Alkyl- oder Arylgruppen mit Akzeptor- beziehungsweise Donatorgruppen und/oder hydrophilen beziehungsweise hydrophoben Gruppen bestehen, oder R¹ und R² ein heterocyclisches System bilden, das seinerseits durch Akzeptor- beziehungsweise Donatorgruppen und/oder hydrophile beziehungsweise hydrophobe Gruppen substituiert ist.

Akzeptorgruppen können -COOR³, -CO-R³, -CN, -SO₂R³ oder - SO₂OR³, mit R³ = Metall, H, Alkyl, Aryl, Ammonium oder funktionalisiertes Alkyl oder Aryl, wie zum Beispiel ω-Carboxyalkyl, HSO₃-CₓH_{y}- H₂N-CₓH_{y}- oder H₂N-SO₂-CₓH_{y}-(x,y=1-45), sein. Donatorgruppen können SR⁴, OR⁴ oder N(R⁴)₂, mit R⁴ = H, Alkyl, Aryl, oder funktionalisiertes Alkyl oder Aryl, sein.
Hydrophile Gruppen können -SO₃M(M=Metall), COOM, -(CH₂-CH₂-O)ₙ R⁴ sein. Hydrophobe Gruppen können Alkyl, Fluoralkyl, Perfluoralkyl, Fluoraryl, Perfluoraryl sein.

Die organischen Gruppen R¹ und R² können:
- substituiert oder unsubstituiert, verzweigt oder unverzweigt sein,
- eine aliphatische Gruppe umfassen, beispielsweise Reste aus Alkanen, Alkenen, Alkoholen, Ethern, Aldehyden, Ketonen, Carbonsäuren, Estern, Kohlenwasserstoffen, Sulfonsäuren, Aminen, Trialkylammonium, Trialkylphosphonium-, Dialkylsulfoniumsalzen,
- eine cyclische Verbindung sein, beispielsweise alicyclische Kohlenwasserstoffe, wie zum Beispiel Cycloalkyle oder Cycloalkenyle, heterocyclische Verbindungen, wie zum Beispiel Pyrrolidinyl-, Pyrrolinyl-, Piperidinyl- oder Morpholinyl-, Arylgruppen, wie zum Beispiel Phenyl-, Naphthyl- oder Anthracenyl-, und Heteroarylgruppen, wie zum Beispiel Imidazolyl-, Pyrazolyl-, Pyridinyl-, Thienyl-, Thiazolyl-, Furyl- oder Indolyl-,
- das heterocyclische System kann Stickstoff, Kohlenstoff oder weitere Heteroatome enthalten und einen drei-, vier-, fünf-, sechs- oder höhergliedrigen Ring bilden, der wiederum durch H, Alkyl- oder Arylgruppen mit Akzeptor- beziehungsweise Donatorsubstituenten oder Teilen von cyclischen Systemen mit Akzeptor- beziehungsweise Donatorsubstituenten und/oder hydrophilen beziehungsweise hydrophoben Gruppen substituiert ist,
- durch weitere funktionelle Gruppen substituiert sein,
- eine chromophore Gruppe oder ein Farbstoff sein,
- geeignete reaktive Verbindungen wie zum Beispiel Triarylammonium-, Triarylphosphonium-, Diarylsulfonium- und Aryliodoniumsalze sein.

Die Gruppen der organischen Verbindungen der allgemeinen Formel 1 können dabei auf die potentiellen Anwendungsgebiete maßgeschneidert sein, da das Reaktionsprinzip beispielsweise sowohl die Einführung von hydrophilen, wie auch von lipophilen Gruppen gestattet. Die Gruppen können auch ionisch, polymer oder für weitere Reaktionen reaktiv sein. Über die Gruppen können unterschiedliche, anwendungstechnisch interessante Eigenschaften des Kohlenstoffmaterials gezielt verändert werden. So kann beispielsweise die Hydrophilie des Kohlenstoffmaterials so weit gesteigert werden, dass das Kohlenstoffmaterial ohne Verwendung eines Netzmittels in wässrigen Medien stabile Dispersionen bildet.

Als Kohlenstoffmaterial kann Ruß, Graphitpulver, Graphitfasern, Kohlenstofffasern, Kohlenstofffibrillen, Kohlenstoffnanoröhren (Carbon Nanotubes), Kohlenstoffgewebe, glasartige Kohlenstoffprodukte, Aktivkohle oder Fullerene eingesetzt werden.

Als Ruß kann Furnaceruß, Gasruß, Channelruß, Flammruß, Thermalruß, Acetylenruß, Plasmaruß, Inversionsruße, bekannt aus DE 195 21 565, Si-haltige Ruße, bekannt aus WO 98/45361 oder DE 196 13 796, oder metallhaltige Ruße, bekannt aus WO 98/42778, Lichtbogenruß und Ruße, die Nebenprodukte chemischer Produktions-prozesse sind, eingesetzt werden.

Das Kohlenstoffmaterial kann durch vorgelagerte Reaktionen aktiviert werden. Es können Kohlenstoffmaterialien, die als Verstärkerfüllstoff in Kautschukmischungen verwendet werden, eingesetzt werden. Es können Farbruße eingesetzt werden. Weitere Kohlenstoffmaterialien können sein: Leitfähigkeitsruß, Kohlenstoffmaterial zur UV-Stabilisierung, Kohlenstoffmaterial als Füllstoff in anderen Systemen als Kautschuk, wie zum Beispiel in Bitumen oder Kunststoff, oder Kohlenstoffmaterial als Reduktionsmittel in der Metallurgie.

Ein weiterer Gegenstand der Erfindung ist ein Verfahren zur Herstellung des erfindungsgemäßen Kohlenstoffmaterials mit organischen Gruppen, welches dadurch gekennzeichnet ist, dass man Kohlenstoffmaterial mit organischen Verbindungen der allgemeinen Formel 1 umsetzt.

Die organische Verbindung der allgemeinen Formel 1 kann auf das Kohlenstoffmaterial aufgebracht werden durch Einmischen oder Aufsprühen. Die organische Verbindung der allgemeinen Formel 1 kann als Pulver, Schmelze oder Lösung aufgebracht werden. Besonders vorteilhaft kann die Aufbringung der organischen Verbindung der allgemeinen Formel 1 während der Herstellung des Kohlenstoffmaterials sein, wobei die Zugabe der organischen Verbindung vorzugsweise an einer Position des Reaktors erfolgt, der die notwendige Temperatur aufweist. Die Reaktion zur Modifizierung des Kohlenstoffmaterials kann vorzugsweise lösungsmittelfrei, aber auch in einem Lösungsmittel, vorzugsweise leicht flüchtigen organischen Lösungsmittel, durchgeführt werden. Die Reaktion zur Modifizierung des Kohlenstoffmaterials kann bei Temperaturen von -80° bis 300°C, vorzugsweise von 80° bis 250°C, durchgeführt werden. Der Energieeintrag kann mittels mechanischer Energie, Schwingungsenergie, beispielsweise Ultraschall, oder Strahlungsenergie, beispielsweise Mikrowellenstrahlung, Wärmestrahlung, Lichtstrahlung, Röntgen- und Elektronenstrahlung, erfolgen.

Die erfindungsgemäßen Kohlenstoffmaterialien mit organischen Gruppen können als Füllstoff, Verstärkerfüllstoff, UV-Stabilisator, Leitfähigkeitsruß oder Pigment in Kautschuk, Kunststoff, Druckfarben, Tinten, Inkjet-Tinten, Lacken und Farben, Bitumen, Beton und anderen Baustoffen oder Papier eingesetzt werden. Ferner können die erfindungsgemäßen Kohlenstoffmaterialien mit organischen Gruppen als Reduktionsmittel in der Metallurgie verwendet werden.

Ein weiterer Gegenstand der Erfindung ist eine Dispersion, welche dadurch gekennzeichnet ist, dass diese das erfindungsgemäße Kohlenstoffmaterial mit organischen Gruppen enthält.

Dabei kann die organische Gruppe auf das jeweilige Dispersionsmedium maßgeschneidert werden. So können mit polaren organischen Gruppen modifizierte Kohlenstoffmaterialien besonders für polare Medien geeignet sein. Polare Medien können Lösungsmittel, wie zum Beispiel Alkohole, Ketone, Ester, Säuren, Amine, Glykole, Glykolether oder halogenierte Lösungsmittel, aber auch Oligomere beziehungsweise Polymere mit polaren Gruppen, wie zum Beispiel Carbonyl-, Ester-, Amino-, Carboxyl- und/oder Hydroxylgruppen, sein. Für wässrige Medien können Kohlenstoffmaterialien mit organischen Gruppen, wie beispielsweise -SO₃X, COOX oder OH, mit X zum Beispiel = H, Alkaliionen oder Ammoniumionen, besonders gut geeignet sein. Für hydrophobe Medien, wie aliphatische, aromatische, heteroaliphatische und/oder heteroaromatische Kohlenwasserstoffe, können hydrophob modifizierte Kohlenstoffmaterialien mit hydrophoben Gruppen, wie Alkyl, Alkyloxy, Aryl und/oder Hetaryl, besonders gut geeignet sein. Für Medien, die hinsichtlich ihrer Polarität zwischen den relativ unpolaren, hydrophoben Medien und den stark polaren Medien liegen, wie zum Beispiel Ether und/oder Gemische aus polaren und unpolaren Medien, können speziell abgestimmte Modifizierungen, zum Beispiel mit Amino-, Carbonyl- oder Halogensubstituenten, besonders gut geeignet sein.

Die erfindungsgemäße Dispersion kann in Druckfarben, Tinten, Lacken und Farben verwendet werden.

Die erfindungsgemäßen Kohlenstoffmaterialien mit organischen Gruppen weisen den Vorteil auf, dass
- polar modifizierte Kohlenstoffmaterialien (zum Beispiel mit -SO₃M-Substituenten) besser in polaren Systemen, vorrangig Wasser, dispergierbar sind,
- unpolar modifizierte Kohlenstoffmaterialien (zum Beispiel mit Alkylgruppen) besser in unpolaren Systemen, wie zum Beispiel Ölen, dispergierbar sind,
- geeignete modifizierte Kohlenstoffmaterialien mit polaren oder sterisch sperrigen Gruppen in den Systemen elektrostatisch, beziehungsweise sterisch stabilisiert werden und zur Stabilisierung keine weiteren Hilfsstoffe wie zum Beispiel Netzmittel notwendig sind,
- nach dem erfindungsgemäßen Verfahren modifizierte Kohlenstoffmaterialien besser in Dispersionen stabilisiert sind und so bessere koloristische Eigenschaften, wie Farbtiefe und Blaustichigkeit, aufweisen,
- Kohlenstoffmaterialien mit gebundenen Farbstoffen veränderte Farbtöne aufweisen,
- Kohlenstoffmaterialien mit weiterhin reaktiven Substituenten zur Kopplung und Vernetzung in Systemen (zum Beispiel Kautschuk) genutzt werden können,
- reaktiv modifizierte Kohlenstoffmaterialien eine Anbindung der Kohlenstoffmaterialien an ein Polymer ermöglichen und
- Kohlenstoffmaterialien dabei arm an Nebenprodukten, Salzen, Säuren und Feuchtigkeit hergestellt werden können.

### Beispiele

Als Kohlenstoffmaterial werden in den Beispielen die Ruße Farbruß FW 1, Farbruß FW 18 und Printex 95 eingesetzt. Die genannten Ruße sind Produkte der Firma Degussa AG.

Die dynamische und statische Oberflächenspannung werden mit dem Blasentensiometer BP2 der Fa. Krüss, die Viskosität mit einem Physica US 200 (Doppelspaltmesssystem) und der pH-Wert mit dem pH-Meter CG 837 gemessen.

### Beispiel 1:

### Modifizierung von Kohlenstoffmaterial mit Natrium-3-(5-mercaptotetrazol-1-yl)benzensulfonat

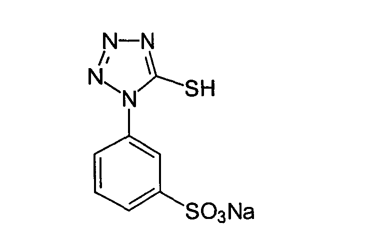

2 g Natrium-3-(5-mercaptotetrazol-1-yl)benzensulfonat werden in 150 ml Wasser gelöst, 10 g Ruß FW 18 hinzugegeben, danach das Lösungsmittel im Vakuum abdestilliert und das verbliebene Gemisch 3 Stunden auf 200 °C erhitzt. Der modifizierte Ruß wird mit 150 ml Wasser gewaschen und danach bei Raumtemperatur getrocknet.

### Beispiel 2:

### Modifizierung von Kohlenstoffmaterial in fester Phase mit Natrium-3-(5-mercaptotetrazol-1-yl)benzensulfonat

2 g Natrium-3-(5-mercaptotetrazol-1-yl)benzensulfonat und 10 g Ruß FW 18 werden gemischt und anschließend 4 Stunden auf 200 °C erhitzt.

### Beispiel 3:

### Modifizierung von Kohlenstoffmaterial in fester Phase mit Natrium-3-(5-benzylthiotetrazol-1-yl)benzensulfonat

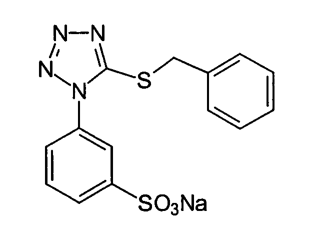

2 g Natrium-3-(5-benzylthiotetrazol-1-yl)benzensulfonat werden in 150 ml Wasser gelöst, 10 g Ruß FW 18 hinzugegeben, danach das Lösungsmittel im Vakuum abdestilliert und das verbleibende Gemisch 3 Stunden auf 200 °C erhitzt. Der modifizierte Ruß wird mit 150 ml Wasser gewaschen und danach bei Raumtemperatur getrocknet.

### Beispiel 4:

### Modifizierung von Kohlenstoffmaterial mit Natrium-3-(5-natriumsulfonatobutylthiotetrazol-1-yl)benzensulfonat

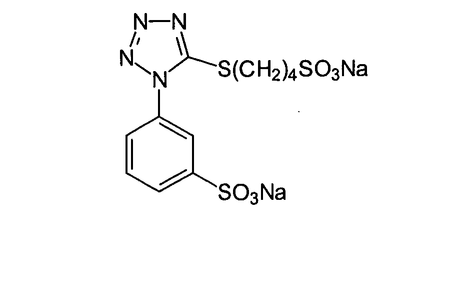

2 g Natrium-3-(5-natriumsulfonatobutylthiotetrazol-1-yl)benzensulfonat werden in 150 ml Wasser gelöst, 10 g Ruß FW 18 hinzugegeben, danach das Lösungsmittel im Vakuum abdestilliert und das verbleibende Gemisch 3 Stunden auf 200 °C erhitzt. Der modifizierte Ruß wird mit 150 ml Wasser gewaschen und danach bei Raumtemperatur getrocknet.

### Beispiel 5:

### Modifizierung von Kohlenstoffmaterial mit 1-(4-Dodecyloxyphenyl)-5-dodecylthiotetrazol

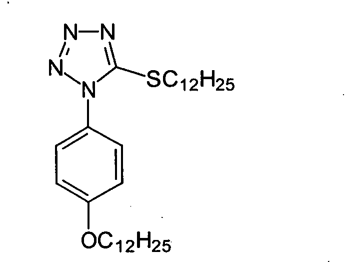

2 g 1-(4-Dodecyloxyphenyl)-5-dodecylthiotetrazol werden in 150 ml Aceton gelöst, 10 g Ruß FW 1 hinzugegeben, danach das Lösungsmittel im Vakuum abdestilliert und das verbleibende Gemisch 3 Stunden auf 200 °C erhitzt. Der modifizierte Ruß wird mit 150 ml Aceton gewaschen und danach bei Raumtemperatur getrocknet.

### Beispiel 6:

### Modifizierung von Kohlenstoffmaterial mit 3-(5-Benzylthiotetrazol-1-yl)benzensulfonamid

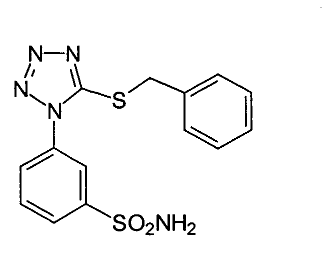

2 g 3-(5-Benzylthiotetrazol-1-yl)benzensulfonamid werden in 150 ml Aceton gelöst, 10 g Ruß Printex 95 hinzugegeben, danach das Lösungsmittel im Vakuum abdestilliert und das verbleibende Gemisch 3 Stunden auf 200 °C erhitzt. Der modifizierte Ruß wird mit 150 ml Aceton gewaschen und danach bei Raumtemperatur getrocknet.

### Beispiel 7:

### Dispersion von modifiziertem Kohlenstoffmaterial in Wasser

15 g Kohlenstoffmaterial mit organischen Gruppen gemäß Beispiel 1 werden mit 85 ml Wasser verrührt und anschließend 30 Minuten bei 5000 U/min mittels Ultra Turrax dispergiert. Die erhaltene Dispersion ist ohne weitere Zugabe von Netzmittel stabil.
Dynamische Oberflächenspannung bei 15 ms: 62 mN/m
Statische Oberflächenspannung bei 3000 ms: 59 mN/m
pH-Wert: 7,5
Viskosität: 2,33 mPas

### Beispiel 8:

### Dispersion von modifiziertem Kohlenstoffmaterial in Wasser

15 g Kohlenstoffmaterial mit organischen Gruppen gemäß Beispiel 2 werden mit 85 ml Wasser verrührt und anschließend 30 Minuten bei 5000 U/min mittels Ultra Turrax dispergiert. Die erhaltene Dispersion ist ohne weitere Zugabe von Netzmittel stabil.
Dynamische Oberflächenspannung bei 15 ms: 65 mN/m
Statische Oberflächenspannung bei 3000 ms: 60 mN/m
pH-Wert: 7,8
Viskosität: 2,17 mPas

### Beispiel 9:

### Dispersion von modifiziertem Kohlenstoffmaterial in Wasser

15 g Kohlenstoffmaterial mit organischen Gruppen gemäß Beispiel 3 werden mit 85 ml Wasser verrührt und anschließend 30 Minuten bei 5000 U/min mittels Ultra Turrax dispergiert. Die erhaltene Dispersion ist ohne weitere Zugabe von Netzmittel stabil.
Dynamische Oberflächenspannung bei 15 ms: 72 mN/m
Statische Oberflächenspannung bei 3000 ms: 65 mN/m
pH-Wert: 8,2
Viskosität: 2,29 mPas

### Beispiel 10:

### Dispersion von modifiziertem Kohlenstoffmaterial in Wasser

15 g Kohlenstoffmaterial mit organischen Gruppen gemäß Beispiel 4 werden mit 85 ml Wasser verrührt und anschließend 30 Minuten bei 5000 U/min mittels Ultra Turrax dispergiert. Die erhaltene Dispersion ist ohne weitere Zugabe von Netzmittel stabil.
Dynamische Oberflächenspannung bei 15 ms: 71 mN/m
Statische Oberflächenspannung bei 3000 ms: 63 mN/m
pH-Wert: 8,0 Viskosität: 2,04 mPas.

## Patentansprüche

1. Kohlenstoffmaterial mit organischen Gruppen, **dadurch gekennzeichnet,**
**dass** dieses erhältlich ist durch die Umsetzung von Kohlenstoffmaterial mit organischen Verbindungen der allgemeinen Formel 1, 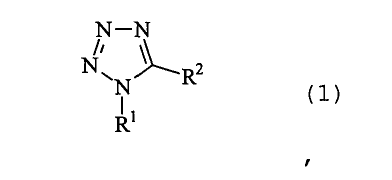 wobei R¹ und R² gleich oder verschieden sind und aus H, Akzeptorgruppen, Donatorgruppen, Alkyl- oder Arylgruppen mit Akzeptor- beziehungsweise Donatorsubstituenten und/oder hydrophilen beziehungsweise hydrophoben Gruppen bestehen oder R¹ und R² ein heterocyclisches System bilden, das seinerseits durch Akzeptor- beziehungsweise Donatorgruppen und/oder hydrophile beziehungsweise hydrophobe Gruppen substituiert ist.

2. Kohlenstoffmaterial mit organischen Gruppen nach Anspruch 1,
**dadurch gekennzeichnet,**
**dass** das Kohlenstoffmaterial Ruß, Graphitpulver, Graphitfasern, Kohlenstofffasern, Kohlenstofffibrillen, Kohlenstoffnanoröhren, Kohlenstoffgewebe, glasartige Kohlenstoffprodukte, Aktivkohle oder Fullerene ist.

3. Kohlenstoffmaterial mit organischen Gruppen nach Anspruch 1,
**dadurch gekennzeichnet,**
**dass** die Akzeptorgruppen -COOR³, -CO-R³, -CN, -SO₂R³ oder -SO₂OR³, mit R³ = Metall, H, Alkyl, Aryl, Ammonium oder funktionalisiertes Alkyl oder Aryl, sind.

4. Kohlenstoffmaterial mit organischen Gruppen nach Anspruch 1,
**dadurch gekennzeichnet,**
**dass** die Donatorgruppen Alkyl, Aryl, SR⁴, OR⁴ oder N(R⁴)₂, mit R⁴ = H, Alkyl, Aryl, oder funktionalisiertes Alkyl oder Aryl, sind.

5. Verfahren zur Herstellung des Kohlenstoffmaterials mit organischen Gruppen nach Anspruch 1,
**dadurch gekennzeichnet,**
**dass** man Kohlenstoffmaterial mit organischen Verbindungen der allgemeinen Formel 1 umsetzt.

6. Verwendung des Kohlenstoffmaterials nach Anspruch 1 als Füllstoff, Verstärkerfüllstoff, UV-Stabilisator, Leitfähigkeitsruß oder Pigment in Kautschuk, Kunststoff, Druckfarben, Tinten, Inkjet-Tinten, Lacken und Farben, Bitumen, Beton und anderen Baustoffen oder Papier.

7. Dispersion,
**dadurch gekennzeichnet,**
**dass** diese Kohlenstoffmaterial mit organischen Gruppen nach Anspruch 1 enthält.

8. Verwendung der Dispersion nach Anspruch 7 in Druckfarben, Tinten, Lacken und Farben.
